# EUROPEAN PATENT APPLICATION

(11) **EP 2 505 213 A2**
(43) Date of publication of application: **03.10.2012**
(21) Application number: 12162042.1
(22) Date of filing: 29.03.2012
(51) Int. Cl.: A61L 15/26, A61L 27/18, A61L 31/06

(54) **System and method for formation of biodegradable ultra-porous hollow fibers and use thereof**

(30) Priority: 30.03.2011 US 201161469306 P; 12.03.2012 US 201213417577
(71) Applicant: Tyco Healthcare Group LP, Mansfield, MA 02048 (US)
(72) Inventor: Gleiman, Seth, Branford, CT Connecticut 06405 (US); Ostapoff, Roland, East Haven, CT Connecticut 06513 (US)
(74) Representative: Soames, Candida Jane

(57) **Abstract**

A system and method for forming biodegradable ultra-porous hollow fibers are disclosed. The fibers are formed by electrospinning a liquid polymer composition (e.g., solution) of a high molecular weight aliphatic polyester in a controlled environment.

## Description

### CROSS-REFERENCE TO RELATED APPLICATION

The present application claims the benefit of and priority to U. S. Provisional Application Serial No. 61/469,306 filed on March 30, 2011, the entire contents of which are incorporated by reference herein.

### TECHNICAL FIELD

The present disclosure relates to a system and method for forming biodegradable ultra-porous hollow fibers and to medical devices formed therefrom.

### BACKGROUND

Polymeric fibers may be formed by many processes within the purview of those skilled in the art, including electrospinning. Electrospinning is an atomization process of a conducting fluid which exploits the interactions between an electrostatic field and the conducting fluid. When an external electrostatic field is applied to a conducting fluid (e.g., a semi-dilute polymer solution or a polymer melt), a suspended conical droplet is formed, whereby the surface tension of the droplet is in equilibrium with the electric field. Electrostatic atomization occurs when the electrostatic field is strong enough to overcome the surface tension of the liquid. The liquid droplet then becomes unstable and a tiny jet is ejected from the surface of the droplet. As it reaches a grounded target, the material can be collected as an interconnected web containing relatively fine, i.e., small diameter, fibers.

There is a continual need to improve the electrospinning process to produce polymeric fibers having a desired diameter, porosity and morphology suitable for use in the medical field.

### SUMMARY

The present disclosure provides biodegradable ultra-porous hollow fibers and medical devices formed therefrom. Specifically, the present disclosure provides highly porous electrospun fibers produced from a polymer composition of an aliphatic polyester and a solvent.

The present disclosure provides a system for forming a medical device. The system includes an environmental chamber comprising an atmosphere having a relative humidity from about 20 % to about 80 % and an electrospinning apparatus disposed within the environmental chamber. The electrospinning apparatus includes at least one reservoir possessing a polymer composition and an ejection tip, the at least one reservoir configured to eject the polymer composition from the ejection tip; a target substrate disposed at a distance from the ejection tip; and an electrical power source coupled to the ejection tip and the target substrate, the electrical power source configured to apply electrical energy to the polymer composition as the polymer composition exits the ejection tip, thereby forming at least one hollow ultra-porous fiber comprising the at least one aliphatic polyester.

The present disclosure also provides a method including providing an electrospinning apparatus comprising at least one reservoir having an ejection tip in an atmosphere having a relative humidity from about 20 % to about 80 % and ejecting a polymer composition from the ejection tip, the polymer composition including at least one aliphatic polyester and at least one solvent. The method also includes applying electrical energy to the polymer composition as the polymer composition exits the ejection tip and recovering at least one hollow ultra-porous fiber comprising the at least one aliphatic polyester.

The present disclosure further provides a method including providing an electrospinning apparatus including at least one reservoir having an ejection tip in an inert atmosphere; adjusting relative humidity of the inert atmosphere to from about 20 % to about 80 %; ejecting a polymer composition from the ejection tip, the polymer composition including at least one aliphatic polyester and at least one solvent; applying electrical energy to the polymer composition as the polymer composition exits the ejection tip; and recovering at least one hollow ultra-porous fiber comprising the at least one aliphatic polyester.

### BRIEF DESCRIPTION OF DRAWINGS

Various embodiments of the present disclosure will be described herein below with reference to the figures wherein:

Fig. 1 is a schematic diagram of a system for forming ultra-porous hollow fibers in accordance with the present disclosure;

Figs. 2A-C are scanning electron microscope images of ultra-porous hollow fibers produced in accordance with the present disclosure;

Fig. 3 is a plot of thermograms obtained of ultra-porous hollow fibers and solution cast films produced in accordance with the present disclosure;

Figs. 4A-C are scanning electron microscope images of ultra-porous hollow fibers produced in accordance with the present disclosure;

Figs. 5A-C are scanning electron microscope images of ultra-porous hollow fibers produced in accordance with the present disclosure;

Fig. 6 shows fiber diameter histograms for ultra-porous hollow fibers produced in accordance with the present disclosure;

Fig. 7A-7D are graphs depicting effects of relative humidity, voltage, flow rate and distance on fiber diameter of ultra-porous hollow fibers produced in accordance with the present disclosure;

Fig. 8 is a set of graphs depicting the interaction between relative humidity and voltage, flow rate and distance on fiber diameter of ultra-porous hollow fibers produced in accordance with the present disclosure; and

Fig. 9 is a scanning electron microscope image of ultra-porous hollow fibers produced in accordance with the present disclosure.

### DETAILED DESCRIPTION

The present disclosure provides a system and method for forming biodegradable ultra-porous hollow fibers. The fibers are formed by electrospinning a liquid polymer composition (e.g., solution) of an aliphatic polyester, which in embodiments may include a high molecular weight aliphatic polyester. In an electrospinning process according to the present disclosure, the polymer composition is supplied through a capillary tube, which is energized by an electrical current that is also applied to a grounded target. The applied voltage induces a charge on the surface of the polymer solution. Mutual charge repulsion on the liquid surface creates a force that counteracts the surface tension of the liquid. As the intensity of the electric field is increased, either through an increase in the applied voltage or a decrease in the distance to the grounded target, the hemispherical surface of the polymer solution at the tip of the capillary tube elongates to form a conical shape known as a Taylor cone. The repulsive electrostatic force may be increased until it is sufficient to overcome the surface tension of the solution, resulting in a charged jet of fluid to be ejected from the tip of the Taylor cone. The discharged polymer solution jet accelerates away from the Taylor cone toward a target substrate, undergoing a bending instability and being subjected to a whipping process (e.g., oscillating of the fibers in a rapid back-and-forth motion) during which the fiber stretches incrementally as any residual solvent evaporates, leaving behind a charged polymer fiber. The fiber impacts the substrate and lays itself in a random configuration over the substrate. An anisotropic non-woven mat may thus be produced by moving the substrate in any pattern and/or direction to align the fibers in a desired configuration along the direction of motion.

In embodiments, the polymer composition for forming ultra-porous hollow fibers may be formed by dissolving a biocompatible polymer in a suitable solvent. Suitable polymers for forming the fibers include polymers such as aliphatic polyesters; polyamides; polyamines; polyalkylene oxalates; poly(anhydrides); polyamidoesters; copoly(ether-esters); poly(carbonates) including tyrosine derived carbonates; poly(hydroxyalkanoates) such as poly(hydroxyvalerate) and poly(hydroxybutyrate); polyimide carbonates; poly(imino carbonates) such as such as poly (bisphenol A-iminocarbonate and the like); polyorthoesters; polyoxaesters including those containing amine groups; polyphosphazenes; poly(propylene fumarates); polyurethanes; polymer drugs such as polydiflunisol, polyaspirin, and protein therapeutics; biologically modified (e.g., protein, peptide) bioabsorbable polymers; and copolymers, block copolymers, homopolymers, blends, and combinations thereof

In some embodiments, aliphatic polyesters may be used including, but not limited to, homopolymers and copolymers of lactide (including lactic acid, D-,L- and meso lactide); glycolide (including glycolic acid); epsilon-caprolactone; p-dioxanone (1,4-dioxan-2-one); trimethylene carbonate (1,3-dioxan-2-one); alkyl derivatives of trimethylene carbonate; Δ-valerolactone; β-butyrolactone; γ-butyrolactone; ε-decalactone; hydroxybutyrate; hydroxyvalerate; 1,4-dioxepan-2-one (including its dimer 1,5,8,12-tetraoxacyclotetradecane-7,14-dione); 1,5-dioxepan-2-one; 6,6-dimethyl-1,4-dioxan-2-one; 2,5-diketomorpholine; pivalolactone; α,α-diethylpropiolactone; ethylene carbonate; ethylene oxalate; 3-methyl-1,4-dioxane-2,5-dione; 3,3-diethyl-1,4-dioxan-2,5-dione; 6,8-dioxabicycloctane-7-one; and combinations thereof.

In embodiments, where the polymer is an aliphatic polyester, the polymer may have a molecular weight of from about 55,000 grams per mole (g/mol) to about 1,000,000 g/mol, in embodiments from about 200,000 g/mol to about 600,000 g/mol, in embodiments from about 250,000 g/mol to about 575,000 g/mol.

Other suitable biodegradable polymers include, but are not limited to, poly(amino acids) including proteins such as collagen (I, II and III), elastin, fibrin, fibrinogen, silk, and albumin; peptides including sequences for laminin and fibronectin (RGD); polysaccharides such as hyaluronic acid (HA), dextran, alginate, chitin, chitosan, and cellulose; glycosaminoglycan; gut; and combinations thereof, As used herein collagen includes natural collagen such as animal derived collagen, gelatinized collagen, or synthetic collagen such as human or bacterial recombinant collagen.

Additionally, synthetically modified natural polymers such as cellulose and polysaccharide derivatives, including alkyl celluloses, hydroxyalkyl celluloses, cellulose ethers, cellulose esters, nitrocelluloses, and chitosan may be utilized. Examples of suitable cellulose derivatives include methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, carboxymethyl cellulose (CMC), cellulose triacetate, and cellulose sulfate sodium salt. These may be collectively referred to herein, in embodiments, as "celluloses."

Suitable solvents for forming a polymer composition according to the present disclosure include polar and non-polar solvents including, but not limited to alcohols, such as, tetrahydrofuran (THF), dimethylformamide (DMF), methanol, ethanol, and/or propanol; halogenated solvents, including chlorinates, fluorinated and brominated hydrocarbons such as hydrofluoroisopropanol (HFIP), dichloromethane, methylene chloride, chloroform, and/or 1, 2-dichloro-ethane; aliphatic hydrocarbons such as hexane, heptene, and/or ethyl acetate; combinations thereof and the like. The polymer composition may be formed by dissolving the polymer in the solvent at a concentration of from about 1 % to about 17 % by weight of the solution, in embodiments from about 2 % to about 45% by weight of the solution.

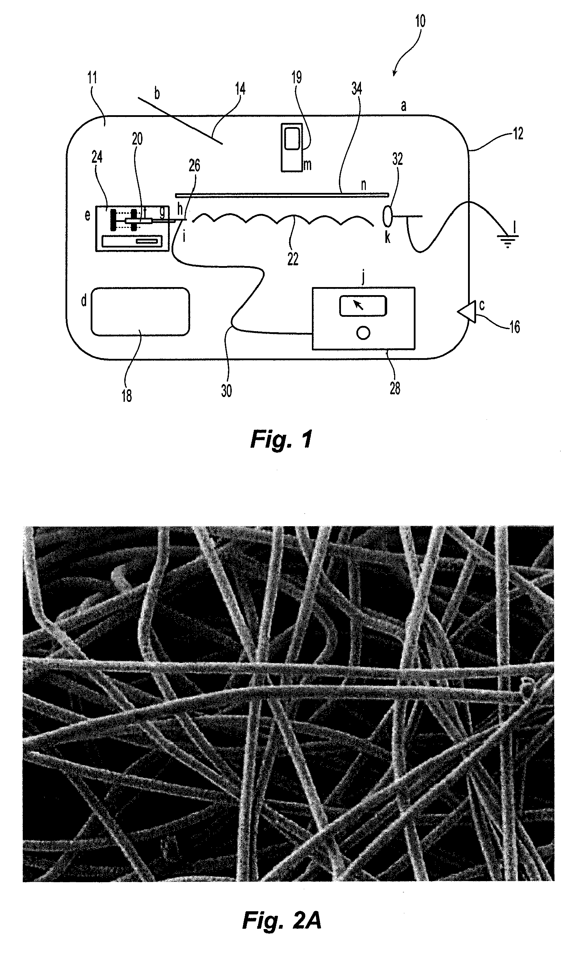

Fig. 1 shows a system 10 for forming ultra-porous hollow fibers according to the present disclosure. The system 10 is setup in an environmental chamber 12, which may be any sealed compartment or container (e.g., glovebox) configured to provide environmental control during the electrospinning process. In accordance with the present disclosure, the atmospheric conditions, such as relative humidity, are controlled to achieve desired fiber porosity and morphology as discussed in further detail below. The environmental chamber 12 may allow one to manipulate the contents stored therein without compromising containment. A portion of the environmental chamber 12 may be transparent or otherwise visually accessible to allow the user to see the contents thereof.

The environmental chamber also includes one or more vents 14 and one or more gas inlets 16. The gas inlets 16 may be coupled to a source of an inert gas or gas mixture. In embodiments, suitable gases include, but are not limited to, nitrogen, carbon dioxide, combinations thereof, and the like. The vents 14 provide for evacuation of the gaseous contents of the chamber 12, including hazardous emissions. In addition, the vents 14, in combination with the gas inlets 16, allow for control of the atmospheric conditions within the chamber 12.

The system 10 also includes a humidity control agent 18 disposed within the chamber 12. The humidity control agent 18 may be any hygroscopic composition suitable to act as a desiccant. In embodiments, the hygroscopic composition may be chemically stable and/or inert with respect to the atmospheric gases within the chamber 12 and/or the polymeric solution used to produce the fibers. The hygroscopic compositions may be a saturated aqueous solution of any suitable salt including, but not limited to, magnesium chloride, sodium bromide, sodium chloride, combinations thereof, and the like. The humidity control agent 18 and/or the atmosphere within the chamber 12 may be adjusted to maintain the relative humidity at levels from about 0 % to about 90 %, in embodiments from about 20 % to about 80 %, in further embodiments from about 25 % to about 35 %;. The system 10 may include a relative humidity meter 19 allowing for measurement and adjustment either manual or automatic based on the measurement of the relative humidity within the chamber 12 to achieve a desired porosity of the hollow fibers as discussed in more detail below.

The system 10 further includes an electrospinning apparatus 11 and one or more reservoirs 20 for storing a fluid, e.g., a polymer solution, from which one or more fibers 22 are to be electrostatically spun. In embodiments, the reservoir 20 may include any suitable type of an ejection mechanism such as a plunger (e.g., syringe). The reservoir 20 is coupled to an ejection pump 24 for controlling the flow rate of the fluid as it is ejected from the reservoir 20. The reservoir 20 may also include a mixer (not shown) for mixing the fluid via physical agitation (e.g., stirring, sonicating, etc.). In embodiments, where the reservoir 20 is a syringe, the ejection pump 24 may be a syringe pump configured to actuate the plunger of the syringe, thereby providing for precise control of the flow rate of the polymer solution.

The reservoir 20 includes a tip 26 for directing the ejected fluid therefrom and forming a stream of fluid having a desired size. In embodiments, the tip 26 may be a cannula, needle, or any other suitable device having a tubular or capillary structure. At least a portion of the tip 26 is formed from a conductive material and is coupled to an electrical power source 28. The power source 28 may be in electrical communication with the tip 26 via a wire 30. The power source 28 may be a direct current power source, configured to supply power in a continuous or pulsatile manner to the tip 26. The applied voltage to the tip 26 from the power supply 28 may be from about 10 kV to about 30 kV, in embodiments from about 15 kV to about 25 kV, in embodiments from about 16 kV to about 24 kV.

During operation, the reservoir 20 ejects the fluid at a predetermined flow rate. The predetermined flow rate will depend upon the polymer solution utilized, the desired morphology of the fibers, and the like. In embodiments, The ejection pump 24 and the reservoir 20 are configured to provide a flow rate from about 1 milliliter per hour (mL/hr) to about 3 mL/hr, in embodiments, from about 1.5 mL/hr to about 2.5 mL/hr, in embodiments from about 1.8 mL/hr to about 2.2 mL/hr. The flow rate of the fluid may also be adjusted to achieve a desired porosity and morphology of the hollow fibers as discussed in more detail below.

Simultaneously, the tip 26 is energized by the power source 28, electrospinning the fluid, as the fluid is ejected from the reservoir 20. Upon being ejected from the reservoir 20 and subjected to electrical energy, the fluid solidifies and forms fibers 22, which are then deposited on a target substrate 32. The substrate 32 is formed from a conductive material and is grounded, either independently or to a ground terminal of the power source 28. The substrate 32 may be a metallic plate of any suitable shape including, but not limited to, rectangular, circular, oval, etc. In embodiments, the substrate 32 may be shaped as a structural support frame for a medical device, such that the electrospun fibers 22 are deposited on the frame to form a fiber-coated medical device.

The substrate 32 may be moved during electrospinning to achieve a desired configuration of the fibers (e.g., anisotropic or isotropic configurations) as the fibers 22 are deposited thereon. In embodiments, the substrate 32 may be rotated about an axis during electrospinning, which allows for the control of the thickness of the fibers (e.g., targeting the edge of a circular substrate 32 that is rotated about its central axis) by adjusting the distance traveled from the tip 26 to the substrate 32. Electrospinning may continue until a desired amount of fibers have been formed, which may be from about 1 minute to about 24 hours, in embodiments from about 30 minutes to about 5 hours.

In embodiments, the substrate 32 may be disposed from the tip 26 at a distance from about 20 cm to about 40 cm, in embodiments from about 25 cm to about 35 cm. The system 10 may also include a ruler 34 that provides for measurement and/or adjustment of the distance between the tip 26 and the substrate 32. In embodiments, ruler 34 may be coupled to the reservoir 20 and the substrate 32 to secure these components to each other, and to maintain the desired distance therebetween.

Without being bound by any particular theory, it is believed that the pores formed in the resulting fibers are a result of a thermodynamic instability caused by the evaporation of solvent from the emergent electrospun solution. Evaporation triggers, depending on the solvent evaporation rate, a spinodal decomposition and phase separation of the polymer and the solvent into polymer-rich and solvent-rich regions, respectively, which transform into pores upon drying. The relative humidity during fiber formation may thus impart pore formation. In particular, the relative humidity restricts solvent evaporation, which allows for coarsening of the phase-separated morphology, along with additional stretching under the influence of the electric field in the whipping region.

In embodiments, fibers may hydrolytically degrade depending on the type of polymer used in fiber formation. After electrospinning, the fibers may be thermally post-treated to modify their strength retention profile due to hydrolytic degradation. In embodiments, the thermal post-treatment may be done at a temperature of from about 70° C to about 170° C, in embodiments from about 80° C to about 100° C.

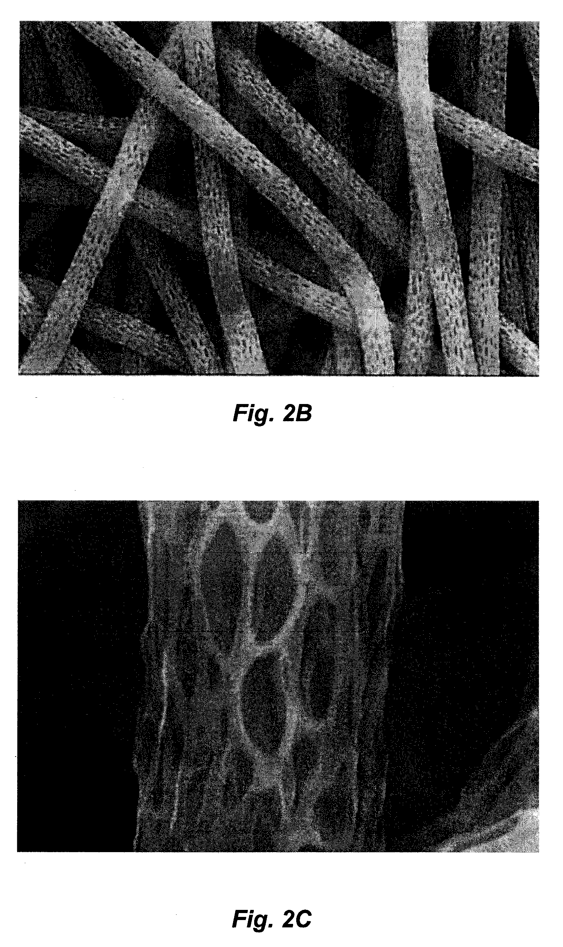

Figs. 2A-C show scanning electron microscope (SEM) images of ultra-porous hollow fibers produced from high molecular weight poly(L-lactic acid) following the methods of the present disclosure at 2,500, 5,210, and 50,000 magnification, respectively. The resulting fibers may have an average effective diameter of from about 10 nanometers (nm) to about 100 micrometers (µm), in embodiments from about 3 µm to about 4.5 µm. Average effective diameter may be calculated by equating an average transverse cross-sectional area of the fibers to a circle having a substantially similar area and then determining the diameter of the circle, which defines the average effective diameter. Fibers may exhibit solid cross sections, hollow fiber morphologies, wrinkled morphologies, thin ribbon-like morphologies, highly porous morphologies, and combinations thereof.

In embodiments, the fibers may include a plurality of pores with a generally elliptical shape, having a major axis and a minor axis. The pores have an average pore length along the major axis of from about 100 nm to about 10 micrometers µm, in embodiments from about 440 nm to about 640 nm, and an average pore width along the minor axis of from about 100 nm to about 10 µm, in embodiments from about 130 nm to about 200 nm. Average effective diameter of the fibers and size of the pores may be tailored by adjusting polymer type and molecular weight, solution conductivity, solvent type, solution viscosity, polymer concentration, relative humidity, tip to substrate distance, and voltage used in electrospinning.

The hollow ultra-porous fibers of the present disclosure may be used to form a variety of medical devices. The medical devices according to the present disclosure may be any structure suitable for being attached or implanted into tissue, including body organs or lumens. Suitable structures include, for example, films, foams, slit sheets, pledgets, tissue grafts, stents, scaffolds, buttresses, wound dressings, meshes, and/or tissue reinforcements. In embodiments, the fibers may be used to form non-woven meshes or tapes, which may be used as passive hemostats. In addition, the non-woven structure of the fibrous mesh lends itself to use as a wound dressing, due to its ability to filter liquids and/or gases.

Medical devices formed from ultra-porous hollow fibers provided in accordance with the present disclosure provide several important advantages over similar electrospun fibers. First, the hollow nature of the fiber allows for a reduced mass of material to be implanted when compared with mats of full-thickness conventional electrospun fibers. Additionally, the highly porous nature of the hollow fiber allows even greater surface area-to-mass ratio. The pores also allow massive loading of the fiber with therapeutic agents, many more times that obtained with conventional fibers. The pores, while too small for cellular infiltration, promote cellular adhesion due to their non-uniform surfaces. The fiber pores increase the porosity of the resulting non-woven mat, thereby allowing one to tailor the cellular interaction of an implant formed with the fibers of the present disclosure.

In embodiments, the medical device and/or fibers of the present disclosure may include one or more therapeutic agents therein. Therapeutic agents may be added to the fibers using any method within the purview of those skilled in the art. Therapeutic agents may be added by applying a solution including the therapeutic agent to the fibers by means including, but not limited to, dipping, spraying, wiping, printing, depositing, coating, and combinations thereof, and the like. In embodiments, the therapeutic agent may be deposited into the lumen of the hollow fibers allowing for the therapeutic agent to elute through the pores. In other embodiments, the fibers may be chopped and/or separated into smaller fibers, which may then be loaded with the therapeutic agents. In yet other embodiments, the therapeutic agent may be included in the polymeric solution that is used to form the fibers, thereby embedding the therapeutic agent within the structure of the fibers and/or medical device as the fibers are formed.

The term "therapeutic agent", as used herein, is used in its broadest sense and includes any substance or mixture of substances that provides a beneficial, therapeutic, pharmacological, and/or prophylactic effect. The agent may be a drug which provides a pharmacological effect. The term "drug" is meant to include any agent capable of rendering a therapeutic affect.

Therapeutic agents may include, for example, amino acids, lipids, lipopolysaccharides, peptides, polypeptides, proteins, polysaccharides, muteins, immunoglobulins, antibodies, cytokines (e.g., lymphokines, monokines, chemokines), blood clotting factors, hemopoietic factors, interleukins (1 through 18), interferons (β-IFN, α-IFN and γ-IFN), erythropoietin, nucleases, tumor necrosis factor, colony stimulating factors (e.g., GCSF, GM-CSF, MCSF), insulin, anti-tumor agents and tumor suppressors, blood proteins, fibrin, thrombin, fibrinogen, synthetic thrombin, synthetic fibrin, synthetic fibrinogen, gonadotropins (e.g., FSH, LH, CG, etc.), hormones and hormone analogs (e.g., growth hormone, luteinizing hormone releasing factor ), vaccines (e.g., tumoral, bacterial and viral antigens), somatostatin, antigens, blood coagulation factors, growth factors (e.g., nerve growth factor, insulin-like growth factor), bone morphogenic proteins, TGF-B, protein inhibitors, protein antagonists, and protein agonists, nucleic acids, such as antisense molecules, DNA, RNA, RNAi, oligonucleotides, polynucleotides, cells, viruses, and ribozymes.

In embodiments, the therapeutic agent may include at least one of the following drugs, including combinations and alternative forms of the drugs such as alternative salt forms, free acid forms, free base forms, pro-drugs and hydrates: anti-adhesives, anesthetics (e.g. local and systemic), antiepileptics, diagnostic agents, cholinomimetics, antimuscarinics, antispasmodics, muscle relaxants, gastrointestinal drugs, diuretics, analgesics/antipyretics (e.g., aspirin, acetaminophen, ibuprofen, naproxen sodium, buprenorphine, propoxyphene hydrochloride, propoxyphene napsylate, meperidine hydrochloride, hydromorphone hydrochloide, morphine, oxycodone, codeine, dihydrocodeine bitartrate, pentazocine, hydrocodone bitartrate, levorphanol, diflunisal, trolamine salicylate, nalbuphine hydrochloride, mefenamic acid, butorphanol, choline salicylate, butalbital, phenyltoloxamine citrate, diphenhydramine citrate, methotrimeprazine, cinnamedrine hydrochloride, and meprobamate), antiasthmatics (e.g., ketotifen and traxanox), antibiotics (e.g., neomycin, streptomycin, chloramphenicol, cephalosporin, ampicillin, penicillin, tetracycline, and ciprofloxacin), antidepressants (e.g., nefopam, oxypertine, doxepin, amoxapine, trazodone, amitriptyline, maprotiline, phenelzine, desipramine, nortriptyline, tranylcypromine, fluoxetine, doxepin, imipramine, imipramine pamoate, isocarboxazid, trimipramine, and protriptyline), antidiabetics (e.g., biguanides and sulfonylurea derivatives), antifungal agents (e.g., griseofulvin, ketoconazole, itraconizole, amphotericin B, nystatin, and candicidin), antihypertensive agents (e.g., propanolol, propafenone, oxyprenolol, nifedipine, reserpine, trimethaphan, phenoxybenzamine, pargyline hydrochloride, deserpidine, diazoxide, guanethidine monosulfate, minoxidil, rescinnamine, sodium nitroprusside, rauwolfia serpentina, alseroxylon, and phentolamine), anti-inflammatories (e.g., (non-steroidal) indomethacin, ketoprofen, flurbiprofen, naproxen, ibuprofen, ramifenazone, piroxicam, (steroidal) cortisone, dexamethasone, fluazacort, celecoxib, rofecoxib, hydrocortisone, prednisolone, and prednisone), antineoplastics (e.g., cyclophosphamide, actinomycin, bleomycin, dactinomycin, daunorubicin, doxorubicin, epirubicin, mitomycin, methotrexate, fluorouracil, gemcitabine, carboplatin, carmustine (BCNU), methyl-CCNU, cisplatin, etoposide, camptothecin and derivatives thereof, phenesterine, paclitaxel and derivatives thereof, docetaxel and derivatives thereof, vinblastine, vincristine, goserelin, leuprolide, tamoxifen, interferon alfa, retinoic acid (ATRA), nitrogen mustard alkylating agents, and piposulfan), antianxiety agents (e.g., lorazepam, buspirone, prazepam, chlordiazepoxide, oxazepam, clorazepate dipotassium, diazepam, hydroxyzine pamoate, hydroxyzine hydrochloride, alprazolam, droperidol, halazepam, chlormezanone, and dantrolene), immunogenic agents, immunosuppressive agents (e.g., cyclosporine, azathioprine, mizoribine, and FK506 (tacrolimus)), antimigraine agents (e.g., ergotamine, propanolol, isometheptene mucate, and dichloralphenazone), sedatives/hypnotics (e.g., barbiturates such as pentobarbital, pentobarbital, and secobarbital, and benzodiazapines such as flurazepam hydrochloride, triazolam, and midazolam), antianginal agents (e.g., beta-adrenergic blockers, calcium channel blockers such as nifedipine, and diltiazem, and nitrates such as nitroglycerin, isosorbide dinitrate, pentearythritol tetranitrate, and erythrityl tetranitrate), antipsychotic agents (e.g., haloperidol, loxapine succinate, loxapine hydrochloride, thioridazine, thioridazine hydrochloride, thiothixene, fluphenazine, fluphenazine decanoate, fluphenazine enanthate, trifluoperazine, chlorpromazine, perphenazine, lithium citrate, and prochlorperazine), antimanic agents (e.g., lithium carbonate), antiarrhythmics (e.g., bretylium tosylate, esmolol, verapamil, amiodarone, encainide, digoxin, digitoxin, mexiletine, disopyramide phosphate, procainamide, quinidine sulfate, quinidine gluconate, quinidine polygalacturonate, flecainide acetate, tocainide, and lidocaine), antiarthritic agents (e.g., phenylbutazone, sulindac, penicillanine, salsalate, piroxicam, azathioprine, indomethacin, meclofenamate, gold sodium thiomalate, ketoprofen, auranofin, aurothioglucose, and tolmetin sodium), antigout agents (e.g., colchicine, and allopurinol), anticoagulants (e.g., heparin, heparin sodium, and warfarin sodium), thrombolytic agents (e.g., urokinase, streptokinase, and alteplase), antifibrinolytic agents (e.g., aminocaproic acid), hemorheologic agents (e.g., pentoxifylline), antiplatelet agents (e.g., aspirin), anticonvulsants (e.g., valproic acid, divalproex sodium, phenytoin, phenytoin sodium, clonazepam, primidone, phenobarbitol, carbamazepine, amobarbital sodium, methsuximide, metharbital, mephobarbital, mephenytoin, phensuximide, paramethadione, ethotoin, phenacemide, secobarbitol sodium, clorazepate dipotassium, and trimethadione), antiparkinson agents (e.g., ethosuximide), antihistamines/antipruritics (e.g., hydroxyzine, diphenhydramine, chlorpheniramine, brompheniramine maleate, cyproheptadine hydrochloride, terfenadine, clemastine fumarate, triprolidine, carbinoxamine, diphenylpyraline, phenindamine, azatadine, tripelennamine, dexchlorphenirarnine maleate, methdilazine, and), agents useful for calcium regulation (e.g., calcitonin, and parathyroid hormone), antibacterial agents (e.g., amikacin sulfate, aztreonam, chloramphenicol, chloramphenicol palirtate, ciprofloxacin, clindamycin, clindamycin palmitate, clindamycin phosphate, metronidazole, metronidazole hydrochloride, gentamicin sulfate, lincomycin hydrochloride, tobramycin sulfate, vancomycin hydrochloride, polymyxin B sulfate, colistimethate sodium, and colistin sulfate), antiviral agents (e.g., interferon alpha, beta or gamma, zidovudine, amantadine hydrochloride, ribavirin, and acyclovir), antimicrobials (e.g., cephalosporins such as cefazolin sodium, cephradine, cefaclor, cephapirin sodium, ceftizoxime sodium, cefoperazone sodium, cefotetan disodium, cefuroxime e azotil, cefotaxime sodium, cefadroxil monohydrate, cephalexin, cephalothin sodium, cephalexin hydrochloride monohydrate, cefamandole nafate, cefoxitin sodium, cefonicid sodium, ceforanide, ceftriaxone sodium, ceftazidime, cefadroxil, cephradine, and cefuroxime sodium, penicillins such as ampicillin, amoxicillin, penicillin G benzathine, cyclacillin, ampicillin sodium, penicillin G potassium, penicillin V potassium, piperacillin sodium, oxacillin sodium, bacampicillin hydrochloride, cloxacillin sodium, ticarcillin disodium, azlocillin sodium, carbenicillin indanyl sodium, penicillin G procaine, methicillin sodium, and nafcillin sodium, erythromycins such as erythromycin ethylsuccinate, erythromycin, erythromycin estolate, erythromycin lactobionate, erythromycin stearate, and erythromycin ethylsuccinate, and tetracyclines such as tetracycline hydrochloride, doxycycline hyclate, and minocycline hydrochloride, azithromycin, clarithromycin), anti-infectives (e.g., GM-CSF), bronchodilators (e.g., sympathomimetics such as epinephrine hydrochloride, metaproterenol sulfate, terbutaline sulfate, isoetharine, isoetharine mesylate, isoetharine hydrochloride, albuterol sulfate, albuterol, bitolterolmesylate, isoproterenol hydrochloride, terbutaline sulfate, epinephrine bitartrate, metaproterenol sulfate, epinephrine, and epinephrine bitartrate, anticholinergic agents such as ipratropium bromide, xanthines such as aminophylline, dyphylline, metaproterenol sulfate, and aminophylline, mast cell stabilizers such as cromolyn sodium, inhalant corticosteroids such as beclomethasone dipropionate (BDP), and beclomethasone dipropionate monohydrate, salbutamol, ipratropium bromide, budesonide, ketotifen, salmeterol, xinafoate, terbutaline sulfate, triamcinolone, theophylline, nedocromil sodium, metaproterenol sulfate, albuterol, flunisolide, fluticasone proprionate, steroidal compounds and hormones (e.g., androgens such as danazol, testosterone cypionate, fluoxymesterone, ethyltestosterone, testosterone enathate, methyltestosterone, fluoxymesterone, and testosterone cypionate, estrogens such as estradiol, estropipate, and conjugated estrogens, progestins such as methoxyprogesterone acetate, and norethindrone acetate, corticosteroids such as triamcinolone, betamethasone, betamethasone sodium phosphate, dexamethasone, dexamethasone sodium phosphate, dexamethasone acetate, prednisone, methylprednisolone acetate suspension, triamcinolone acetonide, methylprednisolone, prednisolone sodium phosphate, methylprednisolone sodium succinate, hydrocortisone sodium succinate, triamcinolone hexacetonide, hydrocortisone, hydrocortisone cypionate, prednisolone, fludrocortisone acetate, paramethasone acetate, prednisolone tebutate, prednisolone acetate, prednisolone sodium phosphate, and hydrocortisone sodium succinate, and thyroid hormones such as levothyroxine sodium), hypoglycemic agents (e.g., human insulin, purified beef insulin, purified pork insulin, glyburide, chlorpropamide, glipizide, tolbutarnide, and tolazamide), hypolipidemic agents (e.g., clofibrate, dextrothyroxine sodium, probucol, pravastitin, atorvastatin, lovastatin, and niacin), proteins (e.g., DNase, alginase, superoxide dismutase, and lipase), nucleic acids (e.g., sense or anti-sense nucleic acids encoding any therapeutically useful protein, including any of the proteins described herein), agents useful for erythropoiesis stimulation (e.g., erythropoietin), antiulcer/antireflux agents (e.g., famotidine, cimetidine, and ranitidine hydrochloride), antinauseants/antiemetics (e.g., meclizine hydrochloride, nabilone, prochlorperazine, dimenhydrinate, promethazine hydrochloride, thiethylperazine, and scopolamine), as well as other drugs useful in the compositions and methods described herein include mitotane, halonitrosoureas, anthrocyclines, ellipticine, ceftriaxone, ketoconazole, ceftazidime, oxaprozin, albuterol, valacyclovir, urofollitropin, famciclovir, flutamide, enalapril, mefformin, itraconazole, buspirone, gabapentin, fosinopril, tramadol, acarbose, lorazepan, follitropin, glipizide, omeprazole, fluoxetine, lisinopril, tramsdol, levofloxacin, zafirlukast, interferon, growth hormone, interleukin, erythropoietin, granulocyte stimulating factor, nizatidine, bupropion, perindopril, erbumine, adenosine, alendronate, alprostadil, benazepril, betaxolol, bleomycin sulfate, dexfenfluramine, diltiazem, fentanyl, flecainid, gemcitabine, glatiramer acetate, granisetron, lamivudine, mangafodipir trisodium, mesalamine, metoprolol fumarate, metronidazole, miglitol, moexipril, monteleukast, octreotide acetate, olopatadine, paricalcitol, somatropin, sumatriptan succinate, tacrine, verapamil, nabumetone, trovafloxacin, dolasetron, zidovudine, finasteride, tobramycin, isradipine, tolcapone, enoxaparin, fluconazole, lansoprazole, terbinafine, pamidronate, didanosine, diclofenac, cisapride, venlafaxine, troglitazone, fluvastatin, losartan, imiglucerase, donepezil, olanzapine, valsartan, fexofenadine, calcitonin, and ipratropium bromide. In some embodiments, the therapeutic agent may be water soluble. In some embodiments, the therapeutic agent may not be water soluble.

The following Examples are being submitted to illustrate embodiments of the present disclosure. These Examples are intended to be illustrative only and are not intended to limit the scope of the present disclosure. Also, parts and percentages are by weight unless otherwise indicated. As used herein, "room temperature" refers to a temperature of from about 20 ° C to about 25° C.

### EXAMPLES

### EXAMPLE 1

Preparation of high molecular weight poly(L-lactic acid) ("PLLA") ultra-porous fibers was as follows.

Approximately 3% by weight/volume solution of PLLA in dichloromethane was prepared and allowed to equilibrate overnight prior to electrospinning. PLLA was obtained from Purac Biomaterials. Dichloromethane solvent (analytical grade 99.99%) for the electrospinning process was acquired from Sigma-Aldrich of St. Louis, MO and used without purification.

Inherent viscosity of the PLLA was measured in chloroform using an Ubbelohde-type viscometer at about 30° C and determined be about 6.74 decaliters per gram (dl/g). The weight average molecular weight (Mw) was determined to be about 566,000 grams per mole (g/mol) using gel permeation chromatography (GPC). Chromatography was performed using a Waters GPC2000 gel permeation chromatography system with two 250 millimeter (mm) x 4.6 mm columns of Polymer Laboratories PL hexafluroisopropanol (HFIP) gel in a series configuration. HFIP was used as the carrier solvent at about 40° C. An integral DAWN™ multi-angle laser light scattering system from Wyatt Technology (Santa Barbara, CA) was used for absolute molecular weight determination. A single point refractive index model supplied by Wyatt Technology's Astra software was also used during molecular weight integration.

A 2.5 milliliter (mL) glass syringe was filled with the polymer solution and mounted on a metered syringe pump from KD Scientific of Holliston, MA. An 18-gauge blunt tip needle was attached to the syringe via polyethylene tubing. The tubing allowed for sufficient distance between the syringe tip and the syringe pump housing, which was capable of grounding the charged polymer solution stream. A high voltage power supply from Gamma High Voltage Research, Inc. of Ormond Beach, FL was attached to the needle with an alligator clip. An approximately 3.8 cm diameter steel washer, wrapped in aluminum foil, was used as a target substrate and was grounded. The entire electrospinning apparatus, including the syringe, the pump, and the power supply, were housed in a glovebox to provide environmental control during the electrospinning operations. Temperature in the glovebox was monitored and recorded, but not controlled. Humidity within the glovebox was regulated using saturated salt solutions of magnesium chloride and sodium bromide (both from Sigma-Aldrich) and dry nitrogen. Relative humidity in the glovebox was allowed to equilibrate for about 24 hours after replacing the saturated salt solutions, and was measured with a dew point/humidity meter from Control Company of Friendswood, TX, mounted to the back of the glovebox.

### EXAMPLE 2

Preparation of low molecular weight PLLA ultra-porous fibers was as follows.

Low molecular weight PLLA ultra-porous fibers were prepared using the process described above with respect to Example 1, using a lower molecular weight PLLA having a Mw of about 250,000 g/mol. The PLLA was dissolved at a higher concentration in dichloromethane of about 5% so that the critical entanglement concentration was held nearly constant (e.g., solution viscosities were approximately equal) and electrospun at 57% RH under the midpoint conditions as defined in Table 1 below.

### COMPARATIVE EXAMPLE 1

Solution casting of high molecular PLLA films was as follows.

The same process was followed as set forth in Example 1 above, to prepare high molecular PLLA solutions. Solutions were poured into a TEFLON® dish and solvent was allowed to evaporate. PLLA film product was collected from the dish.

### EXAMPLE 4

Testing effects of various parameters on high molecular weight PLLA ultra-porous fibers.

A four-level, three-factor, multifactorial set of experiments were performed to test the effect the flow rate (Q, milliliters/hour (mL/hr)), applied voltage (V, kV), distance to target (d, centimeters (cm)), and relative humidity (RH, %) had on fiber diameter and pore size. Factors and levels are listed in Table 1 below.

**Table1**

| Factor (notation, units) | Levels | | |
|---|---|---|---|
| Flow rate (Q, milliliters/hour (mL/hr)) | 1.8 | 2.0 | 2.2 |
| Applied voltage (V, kV) | 16 | 20 | 24 |
| Distance to target (d, centimeters (cm)) | 25 | 30 | 35 |
| Relative humidity (RH, %) | 0 | 35 | 37 |

Electrospinning processes were allowed to proceed at each factor and level combination for up to about 30 minutes, or for sufficient time to ensure complete coverage of the target with electrospun fibers. These fibers were removed from the target and mounted on scanning electron microscope (SEM) stubs with carbon tape. The samples were sputter coated with a gold and palladium mixture from Quorum Technologies of West Sussex, UK, and imaged with an EVO™ LS 15 SEM from Carl Zeiss of Thornwood, NY. Images were post-processed to determine fiber diameter and pore size using ImagJ software available from the National Institutes of Health of Washington D.C. Differential scanning calorimetry (DSC) was performed on select specimens using a Thermal Analysis DSC Q100 calorimeter from TA Instruments of New Castle, DE. DSC samples were loaded into hermetically-sealed aluminum pans and subjected to a heat/cool/heat protocol to temperatures of from about 0° C to about 200° C with heat rates of about 10° C per minute and cool rates of about 20° C per minute. Sample crystallinity was calculated based on the heat of fusion of 100% crystalline PLLA, which was about 93.6 Joules per gram.

Non-woven mats, including micron-sized fibers, were created during the electrospinning process at each combination of factors and levels. Under natural light, the mats appeared white against the silver aluminum foil target. Under all conditions, fibers were found to be concentrated at the target center, with fewer fibers found towards the outer edge of the target.

Molecular weight of the electrospun fibers was measured to ensure that the electrospinning process did not cause molecular weight degradation. Using the same protocol described above, the Mw of electrospun fibers was found to be approximately 512,000 g/mol, indicating that the electrospinning process caused negligible polymer chain degradation during acceleration and orientation in the field and deposition on the target.

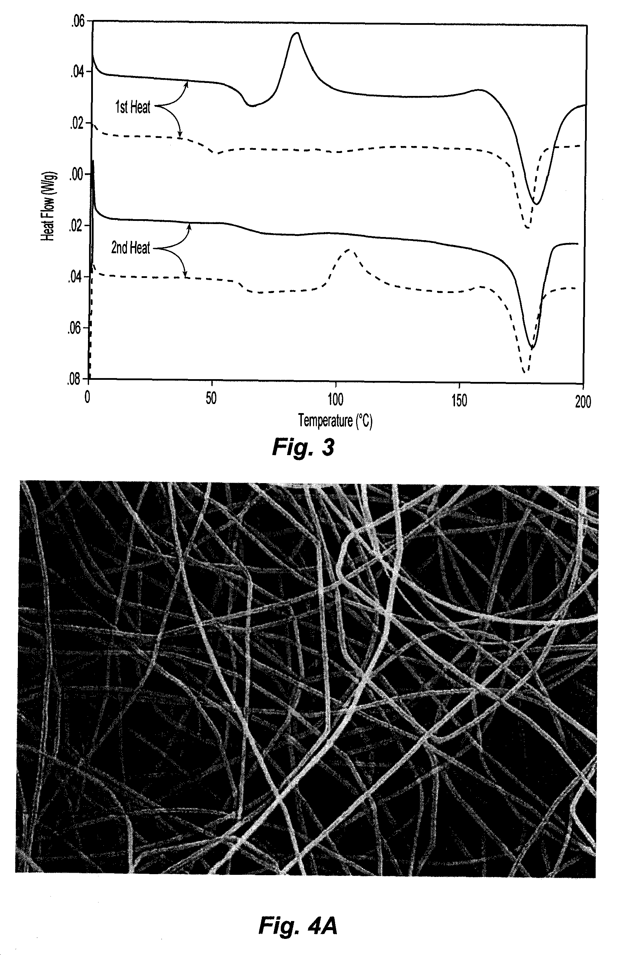

Owing to the unique multiscale physics associated with the electrospinning process, the effect of the electrospinning process on polymer crystallization was measured using DSC and compared with crystalline properties of solution-cast films of Comparative Example 1. These results are shown in Fig. 3 and listed in Table 2 below.

Fig. 3 shows thermograms of the PLLA electrospun fibers of Example 1 (as solid lines) and PLLA solution-cast films of Comparative Example 1 (as dashed lines), including first and second heat cycles. Thermograms were obtained by differential scanning calorimetry (DSC).

Compared to the cast film, during the first heat cycle, the electrospun sample exhibited a large heat flow at the glass transition temperature (Tg) of the polymer, followed immediately by an enthalpy recovery. In addition, the electrospun sample showed a large cold crystallization peak. The cast film exhibited a slight enthalpy recovery but exhibited cold crystallization after the glass transition. The lower Tg of the cast film specimen is believed to be due to the presence of unevaporated solvent that was entrained in the film sample, which plasticized the polymer. The Tg of the cast film of Comparative Example 1 recovered to the nominal value during the second heat cycle.

The crystal perfection, e.g., joining of smaller crystals and polymer chains to join larger, more stable crystals, of the electrospun sample may indicate additional lamellar thickening during the first heat cycle, compared to what was observed with the cast film sample.

**Table 2**

| Processed Specimen | T_{g} (°C) | ΔQ @ T_{g} (W/g) | T_{g} span (°C) | AH @ cold crystallization (J/g) | Tₚₑₐₖ @ cold crystallization (°C) | ΔH @ crystal perfection (J/g) | Tₚₑₐₖ @ crystal perfection (°C) | ΔH @ crystal melt (J/g) | Tₚₑₐₖ @ crystal melt (°C) |
|---|---|---|---|---|---|---|---|---|---|
| Electrospun Specimen of Example 1 (1^{st} heat cycle) | 61.2 | 0.095 | 3.44 | 21.22 | 82.52 | 3.64 | 157.27 | 30.64 | 180.58 |
| Electrospun Specimen of Example 1 (2^{nd} heat cycle) | 63.8 | 0.047 | 10.76 | -- | -- | -- | -- | 35.23 | 180.00 |
| Solvent-cast film of Comparative Example 1(1^{st} heat cycle) | 46.8 | 0.057 | 7.03 | -- | -- | -- | -- | 16.71 | 177.36 |
| Solvent-cast film of Comparative | 62.0 | 0.050 | 4.98 | 16.58 | 105.52 | 1.85 | 159.67 | 15.45 | 177.65 |
| Example 1 (2^{nd} heat cycle) | | | | | | | | | |

Table 2 shows PLLA thermal transitions measured during DSC scans for electrospun fibers and solvent cast film specimens. ΔQ measures the change in heat flow during the glass transition. Tg span is the width of the glass transition temperature. Enthalpy calculations were made through integration of a linear baseline.

Total crystallinities measured during the first heat cycle (taking into account the cold crystallization and crystal perfection of the electrospun sample that occurred during heating, compared to the cast film sample) were about 6% and about 18%, respectively. The polymer chains in the electrospun fibers were aligned primarily by drawing and were then quickly frozen in place by the evaporation of solvent from the fibers. During the cooling cycle in the DSC, the chain alignment in the electrospun fibers appeared to persist within the melt pool.

The broad glass transition temperature observed during the second heat cycle in the electrospun sample (compared to the first heat cycle) suggests a wide distribution of amorphous chain conformations. These conformations were restricted by the elongated extended fiber morphology resulting from the electrospinning process. During reheating of the electrospun specimen, a long melt endotherm was observed until the peak of about 180° C, which suggests a broad distribution of crystal sizes related to the electrospinning process. Total crystallinity in the electrospun fibers approached about 38% upon reheating. Electrospun samples were also subjected to annealing at about 100° C for about 6 hours, with no tension applied, in order to maximize fiber crystallinity, which was about 43%. No changes in fiber morphology were found after the annealing.

The cast film sample exhibited cold crystallization and crystal perfection exotherms during the second heat cycle. It was observed that, contrary to what was observed with electrospinning, the polymer chain alignment within the cast film was not enhanced by stretching. Further, the cooling rate of about 20° C per minute appeared to exceed the crystallization rate such that the melt was effectively quenched, resulting in incomplete crystallization. Crystallization proceeded once heated above the Tg, as evidenced by the peaking of the cold crystallization and crystal perfection. Relative humidity was not shown to have an effect on fiber crystallinity.

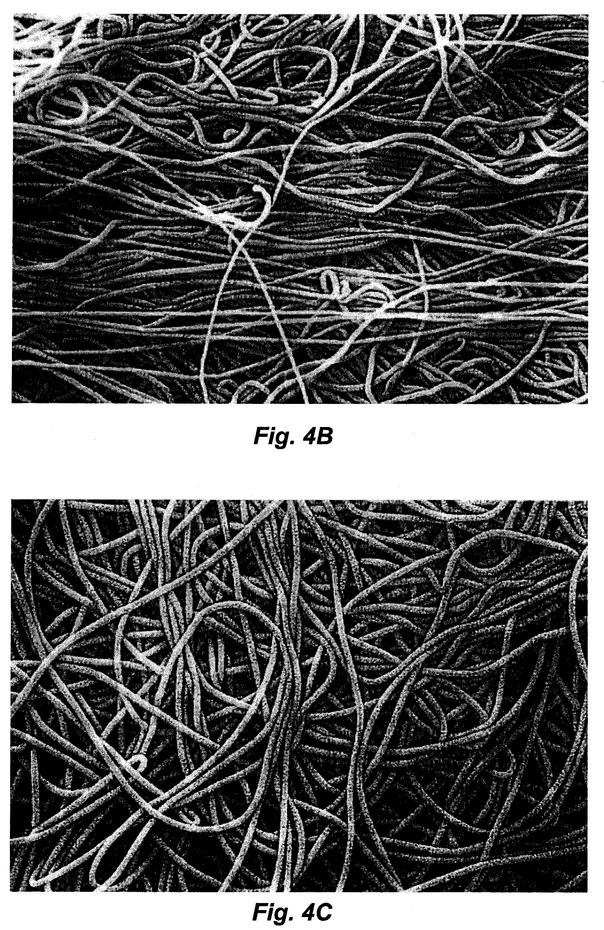

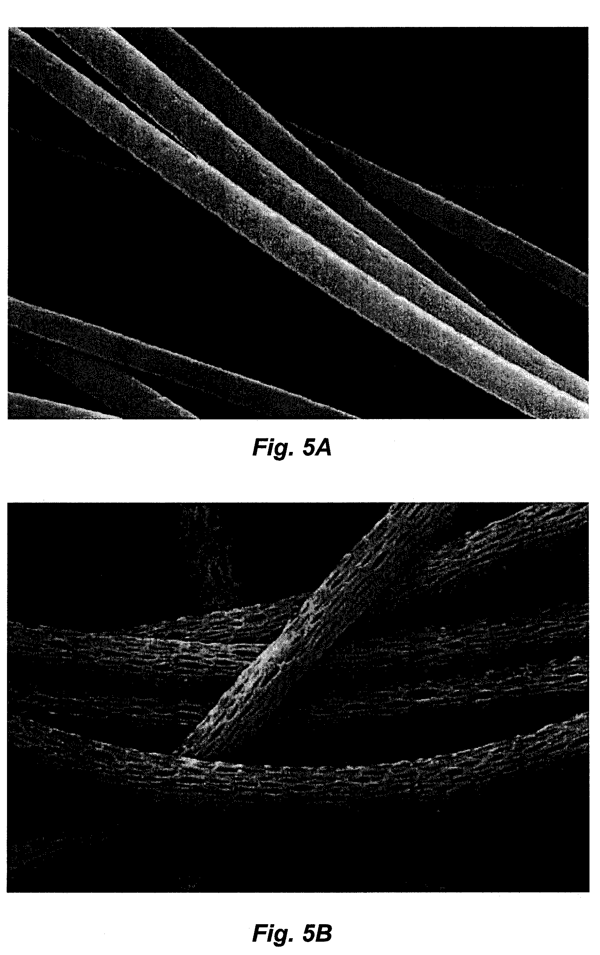

Images of fibers from the midpoint spinning conditions (e.g., Q was about 2 mL/hr, V was about 20 kV, d was about 30 cm, and RH was about 0 %, 35 %, 57 %) are shown in Figs. 4A-C and 5A-C, and are representative of all spinning conditions tested. Figs. 4A-C show SEM images at 1,000x magnification of electrospun PLLA fiber mats processed under the following conditions: Q was about 2 mL/hr; V was about 20 kV; d was about 30 cm; and RH was about 0 %, 35 %, 57 %, respectively. Figs. 5A-C show SEM images at 10,000x magnification of electrospun PLLA fiber mats processed under the following conditions: Q was about 2 mL/hr; V was about 20 kV; d was about 30 cm; and RH was about 0 %, 35 %, 57 %, respectively.

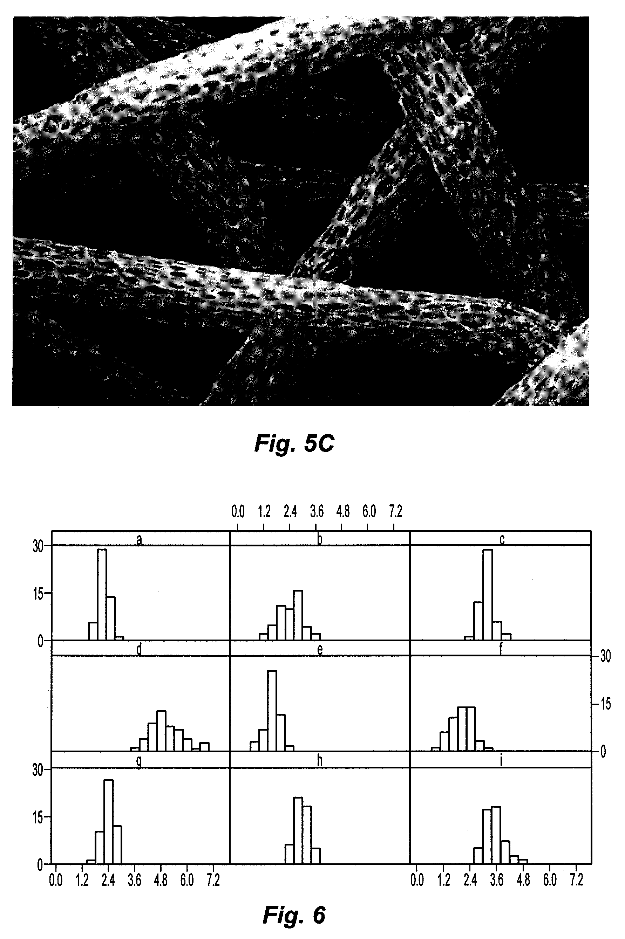

Of particular note was the difference in fiber morphology found under elevated relative humidity spinning conditions, compared to a dry spinning environment. Pores similar to those seen in Figs. 5B and C were found on all fibers spun under all combinations of conditions (e.g., flow rate, applied voltage, distance to target) in humid environments. Without being bound by any particular theory, pore formation may have resulted from a complex interrelationship between simultaneous solvent evaporation out of the electrospun fiber and the resultant spinodal decomposition and phase separation that occurred within the fiber.

SEM images analysis indicated that all combinations of spinning conditions resulted in fiber formation without the presence of beaded fibers or large globules populating any non-woven fiber mat. There was no evidence of an electrospraying effect under any spinning condition. Some conditions resulted in more densely packed mats in which individual fibers were more compliant or wet upon impact with the target, with the individual fibers being always identifiable. At least about 2.5 entanglements per chain were present to ensure complete, stable fiber formation for all combinations of flow rate, applied voltage, distance to target and relative humidity.

The dramatic effect of the relative humidity on fiber morphology is clear from the examination of SEM images of Figs. 5A-C and fiber dimensions as shown in Fig. 6 and Table 3. Fig. 6 shows fiber diameter histograms for nine (9) electrospun samples, a - i, formed under the following conditions (RH, Q, V, d): a) RH = about 0 %, Q = about 2 mL/hr, V = about 20 kV, d = about 30 cm; b) RH = about 35 %, Q = about 2 mL/hr, V = about 20 kV, d = about 30 cm; c) RH = about 57 %, Q = about 2 mL/hr, V = about 20 kV, d = about 30 cm; d) RH = about 57 %, Q = about 2.2 mL/hr, V = about 16 kV, d = about 30 cm; e) RH = about 35 %, Q = about 2.2 mL/hr, V = about 24 kV, d = about 35 cm; f) RH = about 35 %, Q = about 1.8 mL/hr, V = about 24 kV, d = about 35 cm; g) RH = about 57 %, Q = about 2 mL/hr, V = about 16 kV, d = about 30 cm; h) RH = about 0 %, Q = about 2.2 mL/hr, V = about 16 kV, d = about 35 cm; and i) RH = about 57 %, Q = about 2 ml/hr, V = about 24 kV, d = about 25 cm.

The pore structures were essentially absent under dry conditions (e.g., at about 0 % RH), although very slight depressions on the fiber surface were discernable that may represent nascent pores as shown in Fig. 5A. At about 3 5% RH, as shown in Fig. 5B, an abrupt transition to massively porous fiber morphology was observed. The pores had a generally elliptical shape with their major axis aligned to the longitudinal fiber axis and the minor axis perpendicular to the fiber axis. There was a wide distribution of pore lengths (major axis) and widths (minor axis) among the fibers. It appeared that the fibers had high surface porosity and that the porosity persisted throughout the fiber so that they were effectively hollow. This is demonstrated by the electrospun fibers formed at about 57% RH, as shown in Fig. 5C, in which the outline of one fiber can be seen through another fiber.

The minimum fiber diameter produced under conditions tested herein was about 1.59 µm and the largest fiber diameter was about 5.19 micrometers (µm) as shown in Table 3 below. Table 3 shows spinning conditions resulting in selected electrospun fiber properties, such as largest and smallest fiber diameter, and widest and most narrow fiber diameter distribution as measured by the distribution kurtosis and skewness. Distribution kurtosis is a measurement of how different a distribution is from a normal distribution: a positive value typically indicates the distribution has a sharper peak than the normal distribution, and a negative value typically indicates the distribution has a flatter peak than the normal distribution. Distribution skewness is a measurement of distribution symmetry: a negative skewness indicates symmetry to the left and a positive skewness indicates symmetry to the right. A zero value does not necessarily indicate symmetry.

**Table 3**

| Relative Humidity (%) | Flow Rate (mL/hr) | Applied Voltage (kV) | Distance to Target (cm) | Average Fiber Diameter (µm) | Diameter Standard Deviation | Distribution Kurtosis | Distribution Skewness |
|---|---|---|---|---|---|---|---|
| 35 | 2.0 | 24 | 35 | 1.57 | 0.35 | 0.14 | 0.04 |
| 57 | 2.2 | 16 | 30 | 5.19 | 0.79 | 0.31 | 0.61 |
| 35 | 1.8 | 24 | 35 | 1.95 | 0.51 | -1.36 | -0.08 |
| 35 | 2.0 | 20 | 35 | 2.17 | 0.41 | -0.05 | 0.39 |
| 35 | 2.2 | 24 | 30 | 3.16 | 0.41 | -0.05 | 0.19 |
| 57 | 2.0 | 16 | 25 | 2.50 | 0.35 | 14.9 | -3.12 |

As can be seen from Table 3, the widest fiber diameter distribution occurred when spinning conditions were as follows: RH was about 35 %, Q was about 1.8 mL/hr, V was about 24 kV, d was about 35 cm. The most narrow fiber diameter distribution occurred when spinning conditions were as follows: RH was about 57%, Q was about 2.0 mL/hr; V was about 16 kV, d was about 25 cm. Analysis of the pore dimensions showed an average pore length and width of 539±101 nm and 166±32 nm, respectively.

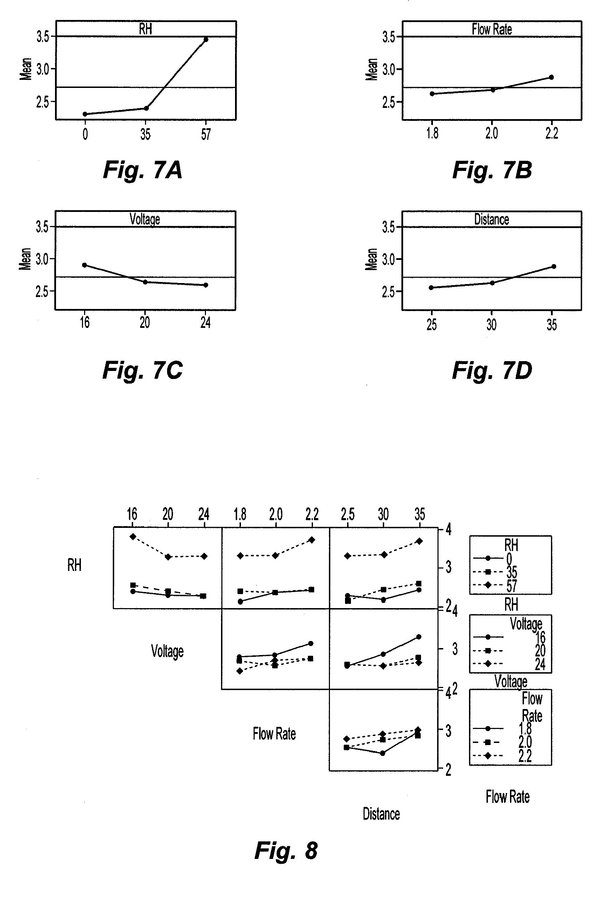

Table 4 below and Figs. 7A-D and 8 describe effects of relative humidity, voltage, flow rate and distance on fiber diameter of ultra-porous hollow fibers. As shown in Figs. 7A-D and 8 and listed in Table 4, a factorial analysis of the spinning condition effects and first level interactions on fiber diameter indicated a strong dependence (p was about 0.000) of fiber diameter on relative humidity, applied voltage and tip-to-target distance. Analysis also indicated less of a dependence (p was about 0.001) on flow rate. Figs. 7A-D show main processing effects on fiber diameter, Fig. 8 shows first-level interaction between relative humidity and voltage, flow rate, and distance on fiber diameter and Table 4 shows p-value (significance) and F-Value (magnitude of significance) of main processing and first level interaction effects on fiber diameter.

**Table 4**

| Process Variable Factor | p-value | F-value |
|---|---|---|
| RH | 0.000 | 192.55 |
| V | 0.000 | 12.97 |
| Q | 0.001 | 7.86 |
| D | 0.000 | 14.19 |
| RH*V | 0.047 | 2.48 |
| RH*Q | 0.038 | 2.61 |
| RH*d | 0.072 | 2.2 |
| V*Q | 0.097 | 2.01 |
| V*d | 0.000 | 5.55 |
| Q*d | 0.027 | 2.84 |

The combination of voltage and distance - or a measure of electric field strength - displayed as the first level interaction term V*d was also found to be a significant factor in affecting fiber diameter although the magnitude of the significance (F was about 5.55) is less than either the primary effect of voltage or distance. All other interactions were determined to be insignificant.

Without being limited by any particular theory, it is believed that the pore formation due to phase separation of the electrospun fibers into polymer-rich and solvent-rich regions was caused primarily by mass loss through solvent evaporation. The mass loss, in turn, drove the subsequent thermodynamic instability and spinodal decomposition. However, humidity played an important rote in stabilizing this evolving system such that the coalescence of solvent-rich regions and growth of an interconnected pore morphology had sufficient time to occur and be deformed during whipping prior to vitrification.

It is believed the humidity created a barrier to mass transfer of solvent out of the fiber at the fiber surface/ambient environment interface, thereby reducing the solvent evaporation rate. The reduction in solvent evaporation rate provided additional time for solvent-rich regions that had nucleated to ripen and coalesce. During this stage, the fiber simultaneously traversed through the bending instability and into the whipping region and was exposed to an additional extensional stress. Since the polymer-rich phase has not vitrified, the application of extensional stress resulted in additional fiber stretching of about 3.25 times in the system, as measured by the final pore aspect ratio assuming an initially isotropic solvent-rich region. During whipping, the solvent evaporation rate increased with fiber surface area and ultimately the polymer vitrified, eliminating additional fiber morphology development.

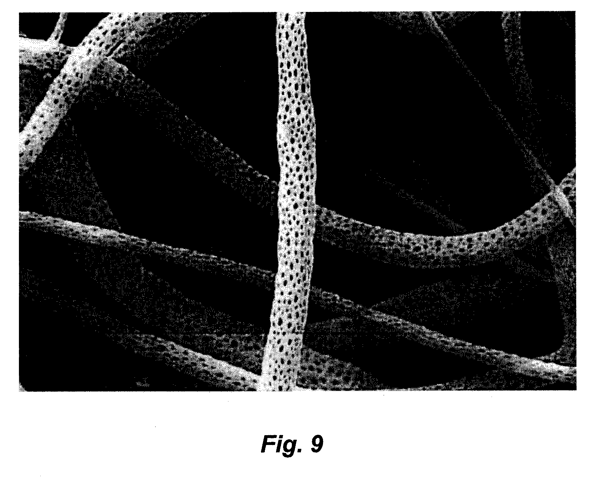

Low molecular weight PLLA ultra-porous fibers of Example 2, as shown in Fig. 9, did not exhibit the dramatic elongated ellipsoidal pore morphology compared to the lower concentration system of high molecular weight PLLA ultra-porous fibers as shown in Figs. 5A-C. Even though the solvent evaporation barrier was the same (constant relative humidity), the higher initial polymer concentration translated the vitrification point upstream of the transition through the bending instability and into whipping region. Solvent-rich regions developed and were frozen in place before coarsening and deforming in the whipping region.

Applying the pore formation model to electrospinning in a dry environment, it was determined that it had no effect on solvent evaporation and the spinodal decomposition was effectively overwhelmed by the kinetic event of ultrafast solvent evaporation. The polymer solution transitioned from a single, stable, solvent-rich phase across the upper critical solution temperature phase diagram to a single, stable, polymer-rich phase faster than the spinodal decomposition could occur.

It will be appreciated that variations of the above-disclosed and other features and functions, or alternatives thereof, may be desirably combined into many other different systems or applications. Also that various presently unforeseen or unanticipated alternatives, modifications, variations or improvements therein may be subsequently made by those skilled in the art which are also intended to be encompassed by the following claims. Unless specifically recited in a claim, steps or components of claims should not be implied or imported from the specification or any other claims as to any particular order, number, position, size, shape, angle, or material.

The invention may be described by reference to the following numbered paragraphs:-
1. A system for forming a medical device, comprising:
   an environmental chamber comprising an atmosphere having a relative humidity from about 20 % to about 80 %; and
   an electrospinning apparatus disposed within the environmental chamber, the electrospinning apparatus comprising:
      at least one reservoir possessing a polymer composition and an ejection tip, the at least one reservoir configured to eject the polymer composition from the ejection tip;
      a target substrate disposed at a distance from the ejection tip; and
      an electrical power source coupled to the ejection tip and the target substrate, the electrical power source configured to apply electrical energy to the polymer composition as the polymer composition exits the ejection tip, thereby forming at least one hollow ultra-porous fiber comprising the at least one aliphatic polyester.
2. The system according to paragraph 1, wherein the at least one aliphatic polyester has a molecular weight of from about 55,000 g/mol to about 1,000,000 g/mol.
3. The system according to paragraph 1, wherein the at least one aliphatic polyester has a molecular weight of from about 200,000 g/mol to about 600,000 g/mol.
4. The system according to paragraph 1, wherein the at least one hollow ultra-porous fiber has an average effective diameter of from about 10 nm to about 100 µm, and includes a plurality of pores having an average pore length of from about 10 nm to about 100 µm and an average pore width of from about 10 nm to about 100 µm.
5. The system according to paragraph 1, wherein the polymer composition includes at least one aliphatic polyester and at least one solvent.
6. The system according to paragraph 1, wherein the at least one aliphatic polyester comprises monomers selected from the group consisting of lactide, glycolide, epsilon-caprolactone, p-dioxanone, trimethylene carbonate, alkyl derivatives of trimethylene carbonate, Δ-valerolactone, β-butyrolactone, γ-butyrolactone, ε-decalactone, hydroxybutyrate, hydroxyvalerate, 1,4-dioxepan-2-one, 1,5,8,12-tetraoxacyclotetradecane-7,14-dione, 1,5-dioxepan-2-one, 6,6-dimethyl-1,4-dioxan-2-one, 2,5-diketomorpholine, pivalolactone, α,α-diethylpropiolactone, ethylene carbonate, ethylene oxalate, 3-methyl-1,4-dioxane-2,5-dione, 3,3-diethyl-1,4-dioxan-2,5-dione, 6,8-dioxabicycloctane-7-one, and combinations thereof
7. A method comprising:
   providing an electrospinning apparatus comprising at least one reservoir having an ejection tip in an atmosphere having a relative humidity from about 20 % to about 80 %;
   ejecting a polymer composition from the ejection tip, the polymer composition including at least one aliphatic polyester and at least one solvent;
   applying electrical energy to the polymer composition as the polymer composition exits the ejection tip; and
   recovering at least one hollow ultra-porous fiber comprising the at least one aliphatic polyester.
8. The method according to paragraph 7, further comprising:
   collecting the at least one hollow ultra-porous fiber at a target substrate disposed at a distance from the ejection tip of from about 25 cm to about 35 cm.
9. The method according to paragraph 7, wherein the at least one ultra-porous fiber has an average effective diameter of from about 10 nm to about 100 µm.
10. The method according to paragraph 9, wherein the average effective diameter of the at least one ultra-porous fiber is from about 3 µm to about 4.5 µm.
11. The method according to paragraph 7, wherein the at least one ultra-porous fiber includes a plurality of pores having an average pore length of from about 10 nm to about 100 µm and an average pore width of from about 10 nm to about 100 µm.
12. The method according to paragraph 7, wherein the electrical energy has a voltage of from about 16 kV to about 24 kV.
13. The method according to paragraph 7, wherein the polymer composition is ejected at a flow rate of from about 1.8 mL/hr to about 2.2 mL/hr.
14. The method according to paragraph 7, wherein the at least one solvent is selected from the group consisting of tetrahydrofuran, dimethylformamide, methanol, ethanol, propanol, hydrofluoroisopropanol, dichloromethane, methylene chloride, chloroform, 1, 2-dichloro-ethane, hexane, heptene, ethyl acetate, and combinations thereof.
15. A method comprising:
   providing an electrospinning apparatus comprising at least one reservoir having an ejection tip in an inert atmosphere;
   adjusting relative humidity of the inert atmosphere to from about 20 % to about 80 %;
   ejecting a polymer composition from the ejection tip, the polymer composition including at least one aliphatic polyester and at least one solvent;
   applying electrical energy to the polymer composition as the polymer composition exits the ejection tip; and
   recovering at least one hollow ultra-porous fiber comprising the at least one aliphatic polyester.
16. The method according to paragraph 15, further comprising:
   collecting the at least one hollow ultra-porous fiber at a target substrate disposed at a distance from the ejection tip from about 25 cm to about 35 cm.
17. The method according to paragraph 15, wherein the at least one ultra-porous fiber has an average effective diameter of from about 10 nm to about 100 µm.
18. The method according to paragraph 17, wherein the average effective diameter of the at least one ultra-porous fiber is from about 3 µm to about 4.5 µm.
19. The method according to paragraph 15, wherein the at least one ultra-porous fiber includes a plurality of pores having an average pore length of from about 10 nm to about 100 µm and an average pore width of from about 10 nm to about 100 µm.
20. The method according to paragraph 15, wherein the electrical energy has a voltage of from about 16 kV to about 24 kV.
21. The method according to paragraph 15, wherein the polymer composition is ejected at a flow rate of from about 1.8 mL/hr to about 2.2 mL/hr.
22. The method according to paragraph 15, wherein the at least one solvent is selected from the group consisting of tetrahydrofuran, dimethylformamide, methanol, ethanol, propanol, hydrofluoroisopropanol, dichloromethane, methylene chloride, chloroform, 1, 2-dichloro-ethane, hexane, heptene, ethyl acetate, and combinations thereof.

## Claims

1. A system for forming a medical device, comprising:
an environmental chamber comprising an atmosphere having a relative humidity from about 20 % to about 80 %; and
an electrospinning apparatus disposed within the environmental chamber, the electrospinning apparatus comprising:
at least one reservoir possessing a polymer composition and an ejection tip, the at least one reservoir configured to eject the polymer composition from the ejection tip;
a target substrate disposed at a distance from the ejection tip; and
an electrical power source coupled to the ejection tip and the target substrate, the electrical power source configured to apply electrical energy to the polymer composition as the polymer composition exits the ejection tip, thereby forming at least one hollow ultra-porous fiber comprising the at least one aliphatic polyester.

2. The system according to claim 1, wherein the at least one aliphatic polyester has a molecular weight of from about 55,000 g/mol to about 1,000,000 g/mol; preferably, wherein the at least one aliphatic polyester has a molecular weight of from about 200,000 g/mol to about 600,000 g/mol.

3. The system according to claim 1 or claim 2, wherein the at least one hollow ultra-porous fiber has an average effective diameter of from about 10 nm to about 100 µm, and includes a plurality of pores having an average pore length of from about 10 nm to about 100 µm and an average pore width of from about 10 nm to about 100 µm.

4. The system according to any preceding claim, wherein the polymer composition includes at least one aliphatic polyester and at least one solvent.

5. The system according to any preceding claim, wherein the at least one aliphatic polyester comprises monomers selected from the group consisting of lactide, glycolide, epsilon-caprolactone, p-dioxanone, trimethylene carbonate, alkyl derivatives of trimethylene carbonate, Δ-valerolactone, β-butyrolactone, γ-butyrolactone, ε-decalactone, hydroxybutyrate, hydroxyvalerate, 1,4-dioxepan-2-one, 1,5,8,12-tetraoxacyclotetradecane-7,14-dione, 1,5-dioxepan-2-one, 6,6-dimethyl-1,4-dioxan-2-one, 2,5-diketomorpholine, pivalolactone, α,α-diethylpropiolactone, ethylene carbonate, ethylene oxalate, 3-methyl-1,4-dioxane-2,5-dione, 3,3-diethyl-1,4-dioxan-2,5-dione, 6,8-dioxabicycloctane-7-one, and combinations thereof

6. A method comprising:
providing an electrospinning apparatus comprising at least one reservoir having an ejection tip in an atmosphere having a relative humidity from about 20 % to about 80 %;
ejecting a polymer composition from the ejection tip, the polymer composition including at least one aliphatic polyester and at least one solvent;
applying electrical energy to the polymer composition as the polymer composition exits the ejection tip; and
recovering at least one hollow ultra-porous fiber comprising the at least one aliphatic polyester.

7. The method according to claim 6, further comprising:
collecting the at least one hollow ultra-porous fiber at a target substrate disposed at a distance from the ejection tip of from about 25 cm to about 35 cm.

8. The method according to claim 6 or claim 7, wherein the at least one ultra-porous fiber has an average effective diameter of from about 10 nm to about 100 µm.

9. The method according to claim 6 or claim 7, wherein the average effective diameter of the at least one ultra-porous fiber is from about 3 µm to about 4.5 µm.

10. The method according to any of claims 6 to 8, wherein the at least one ultra-porous fiber includes a plurality of pores having an average pore length of from about 10 nm to about 100 µm and an average pore width of from about 10 nm to about 100 µm.

11. The method according to any of claims 6 to 10, wherein the electrical energy has a voltage of from about 16 kV to about 24 kV.

12. The method according to any of claims 6 to 11, wherein the polymer composition is ejected at a flow rate of from about 1.8 mL/hr to about 2.2 mL/hr.

13. The method according to any of claims 6 to 12, wherein the at least one solvent is selected from the group consisting of tetrahydrofuran, dimethylformamide, methanol, ethanol, propanol, hydrofluoroisopropanol, dichloromethane, methylene chloride, chloroform, 1, 2-dichloro-ethane, hexane, heptene, ethyl acetate, and combinations thereof.

14. A method comprising:
providing an electrospinning apparatus comprising at least one reservoir having an ejection tip in an inert atmosphere;
adjusting relative humidity of the inert atmosphere to from about 20 % to about 80 %;
ejecting a polymer composition from the ejection tip, the polymer composition including at least one aliphatic polyester and at least one solvent;
applying electrical energy to the polymer composition as the polymer composition exits the ejection tip; and
recovering at least one hollow ultra-porous fiber comprising the at least one aliphatic polyester.

15. The method according to claim 14, further comprising any one or more of the features of the method of claims 7 to 13.
